## Europäisches Patentamt

## European Patent Office

## Office européen des brevets

(19)

(11) Veröffentlichungsnummer: **0 075 215**
**B1**

(12) **EUROPÄISCHE PATENTSCHRIFT**

(45) Veröffentlichungstag der Patentschrift:
05.02.86

(51) Int. Cl.⁴: **C 12 Q 1/18**

(21) Anmeldenummer: **82108345.8**

(22) Anmeldetag: **10.09.82**

(54) Vorrichtungen zum Nachweis von Mikroorganismen hemmenden Stoffen.

(30) Priorität: **16.09.81 DE 3136695**

(43) Veröffentlichungstag der Anmeldung:
**30.03.83 Patentblatt 83/13**

(45) Bekanntmachung des Hinweises auf die Patenterteilung:
**05.02.86 Patentblatt 86/6**

(84) Benannte Vertragsstaaten:
**AT BE CH DE FR GB IT LI LU NL SE**

(56) Entgegenhaltungen:
**DE - A - 2 825 636**
**GB - A - 1 563 088**

**KONTAKTE (MERCK), Nr. 3, 1980 Darmstadt J. KLEIN "Trägerfixierung von Mikroorganismen in polymer Matrix" Seiten 29 bis 36**

(73) Patentinhaber: **Boehringer Mannheim GmbH, Sandhofer Strasse 116, D-6800 Mannheim 31 (DE)**

(72) Erfinder: **Wielinger, Hans, Dr., Im Langgewann 7, D-6940 Weinheim (DE)**
Erfinder: **Wieczorek, Lothar, Dr. rer. nat., Lessingstrasse 9, D-6941 Laudenbach (DE)**
Erfinder: **Bielsteiner, Manfred, Wallstadter-Strasse 46, D-6800 Mannheim 51 (DE)**

LIBER, STOCKHOLM 1986

**Beschreibung**

Die Erfindung betrifft Vorrichtungen zum Nachweis von Mikroorganismen hemmenden Stoffen.

Der Nachweis von Antibiotika und anderen, das Wachstum von Mikroorganismen hemmenden Stoffen, wird allgemein nach dem Prinzip der Agardiffusion (z-B. Blättchentest, Agarlochtest) durchgeführt. Als Indikatororganismen werden im allgemeinen Mikroorganismen aus der Familie der Bacillaceae eingesetzt, deren ausgeprägte Morphogenese für den Bio-Assay ausgenutzt wird. Der Fähigkeit, dieser Bakterien zur Endosporenbildung kommt eine große ökologische und anwendungstechnische Bedeutung zu, da sie eine erhöhte Resistenz gegenüber Umwelteinflüssen wie Hitze, Trockenheit, toxische Substanzen und Strahlung erreichen. In diesem kryptobiotischen Zustand persistieren die Endosporen solange, bis günstige Milieubedingungen eine Sporenkeimung und folgendes vegetatives Wachstum zulassen. Auf der anderen Seite kann die relativ hohe Sensitivität gegenüber wachstumshemmenden Substanzen (Antibiotika, Detergenzien, Stoffwechselantagonisten usw.) während der Keim- beziehungsweise vegetativen Phase zum qualitativen und quantitativen Nachweis dieser Stoffe herangezogen werden. Der Einsatz von Bakterien aus der Familie der Bacillaceae als Bioindikatoren zum Nachweis wachstumshemmender Substanzen wird heute routinemäßig betrieben. Dazu werden Endosporen dieser Bakterien in einen Nähragar eingebettet und die zu untersuchende Substanz nach dem Prinzip der Agardiffusion getestet.

In der Humanmedizin wird die Diagnose bakterieller Infektionskankheiten in Körperflüssigkeiten oft durch die Gegenwart von Hemmstoffen erschwert. Der Anteil an falsch negativen dussagen aufgrund einer Hemmwirkung kann zum Beispiel in der Harndiagnostik bis zu 30% der zu untersuchenden Urine umfassen. Aus diesem Grunde wird parallel zum routinemäßigen Bakteriennachweis ein Test auf Hemmstoffgegenwart gefordert, der aber wegen seines komplizierten Ablaufs für Routineuntersuchungen in der Regel zu aufwendig ist und daher sehr selten durchgeführt wird. Bei einem solchen Test müssen Endosporen in Petrischalen in einen Nähragar eingebettet werden, auf diesen Filterblättchen, die mit der Probe getränkt und getrocknet sind, aufgelegt, bei niedrigen Temperaturen vorinkubiert und bebrütet werden. Bei Anwesenheit von Hemmstoffen unterbleibt um das entsprechende Filterblättchen das Wachstum der Bakterien. Neben der komplizierten Handhabung ist der Einsatz dieser Methode auch wegen der bei solchen Verfahren relativ schwierigen Probenidentifizierung nicht unproblematisch und außerden ist das Verfahren bei kleinen Probenzahlen unrationell. Dazu kommt noch die sehr geringe Lagerstabilität der mit Sporen versetzten Agarplatten; sie sind nur wenige Wochen bei Kühlschranklagerung haltbar.

In der Milch- und Fleischwirtschaft ist eine Überprüfung der Produkte auf Antibiotikafreiheit verbindlich vorgeschrieben. Hierfür wird als Indikatormikroorganismus vorzugweise Bacillus stearothermophilus var. calidolactis eingesetzt.

Die Untersuchung der Proben erfolgt in der gleichen aufwendigen Art und Weise wie oben für Harnproben beschrieben. Die Aufgabenstellung der vorliegenden Erfindung war es, ein Fixier- beziehungsweise Einschlußverfahren für lebende Zellen zu finden, bei dem die Zellen während des Einschlußverfahrens nicht geschädigt werden, ihre duskeim- und Teilungsfähigkeit voll erhalten bleibt, die Sensibilität auskeimender Sporen gegenüber Antibiotika nicht wesentlich beeinflußt wird und die eingeschlossenen Zellen mit der Einschlußmatrix technisch problemlos zu verarbeiten sind.

Die Zellen müssen also während der Lagerung im eingeschlossenen Zustand potentiell wachstumsfähig bleiben und das Wachstum erst durch einen von außerhalb der Matrix gesetzten Reiz induziert werden. Das Stützgerüst der Matrix darf das Zellwachstum nicht wesentlich einengen. Für das Einschlußverfahren dürfen keine Reagenzien verwendet werden, die mit den Zellen reagieren, oder solche, die auf das Wachstum der Zellen schädigend wirken könnten.

Zum Trägerfixieren von Mikroorganismen sind verschiedene Verfahren bekannt, die sich in folgende Kategorien einteilen lassen: Adsorption an einer Oberfläche, kovalente Bindung an einer Oberfläche, Vernetzung der Zellen untereinander, Einkapseln der Zellen, Einschließen der Zellen in eine poröse Matrix. (Vergleiche J. Klein, Kontakte 3/80 Firmenzeitschrift der Firma E. Merck, Darmstadt).

Die Verfahren zielen alle darauf ab, das enzymatische Potential der Zellen auf möglichst engen Raum zu konzentrieren und zu fixieren, um die Enzymaktivitäten in einer Art Käfig für Umsatzreaktionen verschiedenster Art zur Verfügung zu haben.

Dieses Ziel wird durch Fixierung und/oder Einschluß in ein polymeres Netzwerk erreicht. Die bisher bekannten Verfahren werden in den folgenden beiden Tabellen dargestellt, die aus der vorstehenden Literatur über nommen wurden:

**Tabelle 1:**

| Bezeichnung | Größe der Ausgangsmoleküle | Vernetzungsreaktion | Beispiel | Begrenzende Faktoren |
|---|---|---|---|---|
| 1. Gelierung | Polymer | unspezifisch | Agar, Collagen, Carrageenan | geringe Zellbeladung und mechanische Stabilität |
| 2. Fällung | Polymer | unspezifisch | Cellulosetriacetat, Polystyrol, Eudragit | unphysiologische Lösungsmittel |
| 3. Ionotrope Gelbildung | Polymer | ionisch | Alginat, Styrol/Maleinsäure, Carboxymethyl-cellulose | empfindlich bei ionischen Substraten und Pufferlösungen |
| 4. Polykondensation | Oligomer | kovalent | Epoxidharze, Polyurethane | Toxizität der funktionellen Gruppen |
| 5. Polymerisation | Oligomer, Monomer | kovalent, kovalent | Polyacrylamid, Polymethacryl-amid | geringe Zellbeladung |

Tabelle 1: Einstufenverfahren zum Einschluß ganzer Zellen in polymere Netzwerke

| Verfahrenskombination | Beispiel |
|---|---|
| 1. Gelierung und Kondensation | Collagen und Glutaral-dehyd<br>Carrageenan und Glut-araldehyd |
| 2. Ionotrope Gelbildung und kontrollierte Trocknung | Alginat/Ca |
| 3. Ionotrope Gelbildung und Polykondensation | Alginat und Epoxid |
| 4. Polykondensation und Poly-merisation | Epoxid und Polymethacryl-amid |

Tabelle 2: Zweistufenverfahren zum Einschluß ganzer Zellen in polymere Netzwerke

Unter Würdigung der Aufgabenstellung ist es offensichtlich daß keines der bisher bekannten Verfahren als Lösungsweg geeignet wäre, da die bekannten Präparationsverfahren entweder aggressive Reagenzien erfordern, die notwendigen Mengen an Zellen nicht eingeschlossen werden können, oder sich aus der Matrix keine technisch problemlos verarbeitbaren Filme herstellen lassen. Die Herstellung von Filmen ist für den Einsatz der Präparationsform als Reagenz die einzige praktikable Möglichkeit, da alle anderen Variationen zu großvolumig und damit zu unhandlich sind.

Die gestellte Aufgabe wurde wie folgt gelöst:

Zellen werden in eine Mischung, bestehend aus einer wäßrigen Kunststoffdispersion und einer wäßrigen Lösung von 'Öffnern', in die noch Zusatzstoffe eingearbeitet sein können, eingerührt. Diese Rohfilmmasse wird nach üblichem Verfahren auf eine Unterlage, zum Beispiel ausgegossen, aufgestrichen , aufgerakelt usw. und änschließend getrocknet. In so hergestellten Filmen sind die Zellen fest fixiert, so daß sie nur durch grobe mechanische Belastung herausgebracht werden können. Die trockenen Filme enthalten Mikroorganismen in einer Menge von 10 - $10^9$ pro g und wasserlösliche oder wasserquellbare, makromolekulare Öffner sowie gegebenenfalls weitere Zusatzstoffe enthält, wobei das Mengenverhältnis von Kunststoff zu Öffner 1000 : 1 bis 1 : 10.

Die Funktionsweise der Dispersionspartikeln und der 'Öffner' läßt sich folgendermaßen erklären:

Durch das Einarbeiten von 'Öffnern', das heißt wasserlöslichen oder wasserquellbaren makromolekularen Substanzen, in Dispersionsfilme, wird die Eigenschaft dieser Filme dahingehend abgewandelt, daß sie befähigt sind, wesentlich mehr Wasser aufzunehmen, als ein Film ohne Öffner. Mit der Fähigkeit, daß Wasser gut aufgenommen wird, ist auch die Tatsache verbunden, daß in den Film wasserlösliche Substanzen, wie Nährstoffe beziehungsweise Hemmstoffe unschwer eindiffundieren können. Die Öffner präformieren in der Kugelpackung der Dispersionspartikel 'offene Stellen', in denen die Zellen eingelagert sind, ohne jedoch das Filmgerüst so zu stören, daß eine dusbildung eines Filmes nicht mehr möglich ist.

Wird nun von außerhalb des Filmes, durch dessen Anfeuchten ein Wachstumsreiz auf die Zellen ausgeübt, so beginnen diese von den 'offenen Teilen' des Filmes ausgehend zu wachsen. Werden gleichzeitig mit den Nährstoffen Hemmstoffe an die Zellen herangeführt, so unterbleibt das Wachstum zu Kolonien. Die ausgebildeten Kolonien können in bekannter Weise mit Hilfe von Indikatoren wie zum Beispiel pH-Indikatoren, Triphenyltetrazoliumsalzen usw. sichtbar gemacht werden.

Arbeitet man hingegen Zellen in Kunststoffdispersionen ein, denen keine Öffner beigefügt sind und bildet Filme aus, so sind die Zellen dicht eingeschlossen. Werden Dispersionen verwendet, die Filme mit einer

4

Wasseraufnahmefähigkeit bilden, so kann nur ein geringes Auskeimen und Kolonienwachstum beobachtet werden, da der Zutritt von Nährstoffen beziehungsweise Hemmstoffen zu stark beeinträchtigt wird, weil solche Filme keine 'offenen Stellen' besitzen.

Bei Dispersionen, die mehr oder minder hydrophobe Filme bilden ist im allgemeinen kein Kolonienwachstum mehr zu beobachten.

Für das erfindungsgemäße Verfahren ist eine Vielzahl von Rohstoffen einsetzbar.

Die Rohstoffe werden unten näher beschrieben. Es besteht lediglich eine wichtige Voraussetzung, daß sie keinen Hemmstoff für die einzusetzenden Zellen darstellen, daß sie nicht mit Hemmstoffen verunreinigt sind und daß sie die Wirkung von Hemmstoffen nicht aufheben.

Eine Prüfung auf Hemmstoff-Freiheit, die für die Rohstoffkontrolle notwendig ist, läßt sich unschwer nach den bekannten klassischen Verfahren, zum Beispiel Agarverdünnungstest, durchführen.

Als Filmbildner lassen sich praktisch alle wäßrigen Kunststoffdispersionen verwenden, wie zum Beispiel Homopolymere aus Vinylacetat, aus Acrylsäureestern, Copolymere aus Vinylacetat und Vinylpropionat, aus Vinylpropionat, Acrylnitril und Acrylsäureestern, aus Vinylacetat und Maleinsäureestern, aus Butadien und Styrol, aus Vinylchlorid und Vinylpropionat usw.

Als Öffner sind prinzipiell alle makromolekularen wasserlöslichen Substanzen einsetzbar. Darunter fallen zum Beispiel wasserlösliche, vollsynthetische Hochpolymere wie Polyvinylpyrrolidone, Polyvinylalkohole, teilverseifte Polyvinylacetate, Copolymere aus Vinylacetat und Vinylpyrrolidon, Polyäthylenglycole, Polyäthylenoxidharze, Acrylsäurepolymerisate, Mischpolymerisate aus Methylvinyläther und Maleinsäureanhydrid usw.. Ebenso wasserlösliche halbsynthetische Produkte und reine Naturprodukte wie zum Beispiel Methylcellulose, Hydroxyäthylcellulose, Hydroxypropylcellulose, Carboxymethylcellulose, Alginate, Gelatine, Polyzucker, Stärken usw. sowie Mischungen dieser Substanzen. Als Zusatzstoffe können verwendet werden: Puffersalze, Neutralsalze, Zucker, Eiweißkörper, Netzmittel, Pigmente, Weichmacher wie zum Beispiel Clycerin, Propylglycol usw. sowie Indikatoren, die das Wachstum der Mikroorganismen anzeigen, wie zum Beispiel pH-Indikatoren, Triphenyltetrazoliumderivate usw..

Die Filmrohmassen werden so hergestellt, daß man in einem Gefäß die Kunststoffdispersion vorlegt, dazu eine Lösung des Öffners und der Zusatzstoffe gibt und unter Rühren zur Rohfilmmasse homogenisiert. Dabei kann das Mischungsverhältnis zwischen Festkörperanteil aus der Dispersion, Festkörperanteil der Öffner und Zusatzstoffen innerhalb großer Grenzen gewählt werden. Die Mischungsverhältnisse sind nach folgenden Gesichtspunkten zu ermitteln:

a) Die aus der Filmrohmasse gebildeten Filme müssen eine in sich geschlossene Einheit bilden, das heißt sie dürfen keine Risse zeigen, ein Phänomen, das weitgehend von den stoffspezifischen Eigenschaften und den Wechselwirkungen von Dispersion und Öffner bestimmt wird.

b) Die Filme müssen ausreichend 'offen' sein, um den Nährstoffen und gegebenenfalls den Hemmstoffen einen ausreichenden Zutritt zu den Zellen zu ermöglichen, ein Phänomen, das ebenfalls weitgehend von den stoffspezifischen Eigenschaften und den Wechselwirkungen von Dispersion und Öffner sowie Zusatzstoffen bestimmt wird.

c) In gewissem Maße wird das Mischungsverhältnis auch von der Viskosität der Dispersionen und der Öffnerlösungen bestimmt. Dabei ist zu beachten, daß die Viskosität der Filmrohmasse selbstverständlich nur so eingestellt sein darf, daß sie technisch weiter verarbeitbar ist. Das heißt, daß zum Beispiel von hochviskosen Öffnern nur weniger eingesetzt werden können als von niederviskosen Substanzen.

Unter Berücksichtigung aller oben genannten Tatsachen können Mischungsverhältnisse, von Dispersions - Festkörper zu Öffner-Festkörper von 1000 : 1 bis 1 : 10 gewählt werden.

Die Konzentrationen der Zusatzstoffe werden entsprechend den jeweiligen einzelnen Bedürfnissen gewählt. Es ist offensichtlich, daß Indikatoren, Weichmacher und Netzmittel in relativ niedrigen Konzentrationen einzusetzen sind, die sich danach richten, ab wann diese Substanzen eine hemmende Wirkung auf die Zellen ausüben beziehungsweise die Filme zu weich oder zu spröde machen. Die zu wählenden Salzkonzentrationen und Konzentrationen der Zucker- und Eiweißkörper dürfen keinen negativen Einfluß auf das Wachstumsvermögen der Zellen ausüben. Ebenfalls ist zu beachten, daß durch die Hilfsstoffe die Stabilität der Filme nicht negativ beeinflußt werden dürfen, das heißt ihre Konzentration eine obere Grenze nicht übersteigen darf.

Vor der Verarbeitung der Filmrohmassen zu den Filmen werden Zellen in Konzentration von $10 - 10^8$ je ml Rohmasse in diese eingerührt. Die Ausbildung der Filme kann nach den bekannten Verfahren, wie Ausgießen in Formen, Beschichten von Trägern, Beschichten von Stützgeweben, die in die Filmmassen eingeschlossen werden usw. vorgenommen werden.

Die bevorzugten Anwendungsgebiete des erfindungsgemäßen Verfahrens sind:

Der Einsatz des Verfahrens zur Herstellung von Lagerund Transportvorrichtungen für Mikroorganismen. Die Zellen werden dazu in einer Filmmatrix eingeschlossen und diese trocken gelagert. Werden die Mikroorganismen benötigt, wo wird der Film mit einer Nährbouillon überschichtet oder in diese eingelegt, bebrütet und so die Mikroorganismen vermehrt. Eine weitere Anwendungsmöglichkeit ist der Einsatz von Sporenfilmen zum Nachweis von Hemmstoffen in biologischem Material im Rahmen von Untersuchungen in der Humanmedizin, der Veterinärmedizin, der Lebensmittelkontrolle usw.. Dazu wird folgendermaßen vorgegangen: Sporen von gegen Hemmstoffe besonders empfindlichen Mikroorganismen wie zum Beispiel die von <u>Bacillus subtilis</u> oder <u>Bacillus stearo- thermophilus</u> werden in eine Filmrohmasse eingearbeitet. Aus diesen Rohmassen wird auf einem transparenten Träger ein Film ausgestrichen, getrocknet und die Filme in eine handliche Größe geschnitten. Der Sporenfilm wird zusammen mit einen Nährkarton in die zu untersuchenden Lösungen, wie Harn, Milch, Fleischsäfte usw. eingetaucht und bebrütet. Bei Abwesenheit von Hemmstoffen keimen die Sporen aus und das folgende Wachstum kann über einen Indikator nachgewiesen werden. Als Indikatoren werden Substanzen eingesetzt, die entweder die Kolonien anfärben, wie zum Beispiel Triphenyltetrazoliumverbindungen oder pH-Indikatoren, die eine durch das Keimwachstum hervorgerufene pHÄnderung anzeigen.

Eine bevorzugte technische Ausführungsform ist die, daß man den gleichen Aufbau wählt wie er bei Teststreifen üblich ist. Dazu befestigt man auf einem Träger, einen Nährkarton und darüberliegend einen auf einem transparenten Träger ausgebildeten Sporenfilm mit Hilfe eines Netzes so, daß die Filmseite des Sporenfilms auf die dem Träger abgewandte Seite des Nährkartons zu liegen kommt.

Als Nährkarton kann ein mit geeigneten Nährstoffen für die jeweiligen Mikroorganismen imprägniertes, saugfähiges Material, vorzugsweise Filterpapier, Glasfaser- oder Kunststoffvlies, oder ein in trockenem Zustand genügend stabiles Gel verwendet werden. Eingetrocknete Agar-Schichten oder ähnliche Substanzen lassen sich nicht verwenden, da sie zu langsam die Flüssigkeit wiederaufnehmen.

Das erfindungsgemäße Verfahren wird anhand von Beispielen näher erläutert.

## Beispiel 1

In diesem Beispiel sollen die breit gefächerten Möglichkeiten der zu verwendenden wäßrigen Kunststoffdispersionen dargestellt werden:

Zur Herstellung einer Trägermatrix, in die Zellen eingeschlossen sind, wird dabei wie folgt verfahren:

In einem Gefäß werden 60 g Kunststoffdispersion vogelegt. Als Dispersion können zum Beispiel folgende handelsübliche 50 - 60 %-ige Dispersionen in Wasser eingesetzt werden:

Homopolymere aus Vinylacetat, aus Acrylsäureestern, Copolymere aus Vinylacetat und Vinylpropionat, aus Vinylpropionat, Acrylnitril und Acrylsäureestern, aus Vinylacetat und Maleinsäureestern, aus Butadien und Styrol, aus Vinylchlorid und Vinylpropionat usw..

Die Dispersionen werden mit 35 g einer 40 %igen Lösung von Polyvinylpyrrolidon in 1/15 m Phosphatpuffer pH 6,5 verrührt, der pH-Wert überprüft und gegebenenfalls nachgestellt. Dann wird die Mischung mit 200 ml sterilem destilierten Wasser verdünnt und $10^3$ - $10^7$ Zellen von zum Beispiel Sporen von <u>Bacillus subtilis</u> je Gramm Dispersion-Öffnermischung zugegeben und verrührt. 0,5 ml der so erhaltenen Rohfilmmasse werden in quadratische Gefäße von 2 cm Kantenlänge pipettiert und getrocknet.

Bringt man auf die so hergestellten Filme 1 ml einer Nährbouillon, die 0,02 % Triphenyltetrazoliumchlorid (TTC) enthält und bebrütet die verschlossenen Gefäße, so zeigen sich die gewachsenen Kolonien auf der Unterseite des Gefäßes je nach Konzentration des Mikroorganismus als rote Punkte bis zu einem dichten homogenen roten Feld.

## Beispiel 2

In diesem Beispiel sollen die Möglichkeiten der verwendbaren Öffner abgehandelt werden:

2a) In einem Gefäß werden 62 g einer Dispersion zum Beispiel eines Copolymeren aus Vinylacetat und Vinylpropionat mit 2oo ml Wasser vorgelegt. Dazu werden 37 g der Lösungen der Öffner gegeben und gut verrührt. Die Öffnerlösungen werden so hergestellt, daß man in einem 1/15 m Phosphatpuffer pH 6,5 die Öffner in den in der Tabelle angegebenen Konzentrationen löst. Bei sauer reagierenden Öffnern werden die Lösungen mit Natronlauge eingestellt.

Tabelle 3 enthält Beispiele für mögliche Konzentrationen der Öffner in der Pufferlösung und Angaben der aus diesen Mischungen im Film entstehenden Mischungsverhältnissen von Festkörper aus der Kunststoffdispersion zu Festkörper aus dem Öffner.

**Tabelle 3**

| Öffner | Konzentration des Öffners in der eingesetzten Lösung in Puffer | Mischungsverhältnis des Feststoff aus der Dispersion zu Feststoff des Öffners im Film |
|---|---|---|
| Stärke | 1 % | 80 : 1 |
| Dextran | 43 % | 2 : 1 |
| Alginat | 2,35 % | 36 : 1 |
| Methylcellulose | 1,5 % | 57 : 1 |
| Methylenhydroxy-äthylcellulose | 2,5 % | 34 : 1 |
| Methylhydroxypro-pylcellulose | 4,0 % | 21 : 1 |
| Hydroxypropyl-cellulose | 4,0 %<br>0,5 % | 21 : 1<br>170 : 1 |
| Hydroxyäthyl-cellulose | 4,0 % | 21 : 1 |
| Polyvinylalkohol | 8,0 % | 10 : 1 |
| Polyäthylengly-kolharz | 15,0 %<br>5,0 % | 5,5 : 1<br>17 : 1 |
| Polyacrylsäure | 1,7 % | 50 : 1 |
| Copolymerisat aus Methyl-vinyläthan und Malein-säureanhydrid | 23 % | 3,7 : 1 |
| Copolymerisat aus Vinylpyrro-lidon und Vinyl-acetat | 15 % | 5,5 : 1 |

In Abwandlung zu Beispiel 2a kann man auch von einer relativ hochprozentigen Öffnerlösung ausgehen, was immer dann möglich ist, wenn der Öffner eine relativ niederviskose Lösung ergibt.

Tabelle 4 gibt diese Möglichkeit wieder.

**Tabelle 4**

| Öffner | Gewichtsanteile von wäßriger Dispersion zu Öffnerlösung | Mischungsverhältnis des Feststoffs aus der Dispersion zu Feststoff des Öffners im Film |
|---|---|---|
| Polyäthylen-glycol 5o % ige Lösung | 5o g : 45o g – 45o g : 5o g | 1 : 9 – 9 : 1 |
| Polyvinyl-pyrrolidon 42 % ige Lösung | 1o g : 9o g – 9o g : 1o g | 1 : 7 – 11 : 1 |
| Carboxymethyl-cellulose 2 % ige Lösung | 8 g : 1oo g | 2 : 1 |

2c) In Abwandlung zu Bespiel 2a können auch Mischungen von verschiedenen Öffnersubstanzen eingesetzt werden.

46 g einer wäßrigen Dispersion des Mischpolymerisates von Vinylacetat und Vinylpropionat, 4,2 g einer 40 %igen Lösung von Polyäthylenglycol in 1/15 m Citratpuffer pH 6,5 , 16,0 g einer 40 %igen Lösung von Polyvinylpyrrolidon in 1/15 m Citratpuffer (pH 6,5), 0,4 g Glycerin und 200 ml Wasser werden zu einer homogenen Masse verrührt.

In die oben beschriebenen Rohfilmmassen werden Sporen von Bacillus subtilis in einer Konzentration von $10^6$ je Gramm Rohfilmmasse eingearbeitet. Von der so hergestellten Sporenfilmrohmasse werden je 0,5 ml in Gefäße von 2 cm Kantenlänge pipettiert und getrocknet. Bringt man auf die so hergestellten Filme 1 ml einer Nährbouillon, die 0,02 %

Triphenyltetrazoliumchlorid enthält und bebrütetdie verschlossenen Gefäße, so zeigen sich die gewachsenen Kolonien als rote Punkte. Das Wachstum unterbleibt, wenn der Nährlösung eine Probe zugemischt ist, die Hemmstoffe, beispielsweise Antibiotica, in entsprechender Konzentration enthält.

**Beispiel 3**

Verfahren zum Nachweis von Antibiotika in der Milch.

50 g einer Dispersion eines Copolymeres aus Vinylacetat und Vinylpropionat, 20 g einer Lösung von 12 g Polyvinylpyrrolidon in 17 ml Wasser, 1 g Glucose und $10^8$ Sporen von Bacillus stearothermo- philus var. calidolactis werden homogen verrührt, anschließend mit 150 ml Wasser verdünnt und mit Natronlauge auf pH 7,0 eingestellt. Von der so hergestellten Filmmasse werden jeweils 0,1 ml in zylindrische Gefäße von 1 cm Durchmesser mit flachem Boden pipettiert und getrocknet. Zur Testung auf Antibiotika in Milch werden Nährkartons, die aus Milchmedium mit dem Indikator Bromkresolblau hergestellt wurden, in die Probe eingetaucht, auf die Filme aufgelegt, die Gefäße dicht verschlossen und bei 60° C bebrütet. Bei Abwesenheit von Hemmstoffen nehmen die Nährkartons eine olivgrüne Farbe an, in Gegenwart der Hemmstoffe bleiben sie auch nach dem Bebrüten bläulich.

Nach diesem Verfahren können Antibiotika über ihre Wirkung als Hemmstoffe nachgewiesen werden. Die wichtigen Antibiotika zum Beispiel Penicillin, Tetracyclin und Chloramphenicol können nach diesem Verfahren ab folgenden Konzentrationen erfaßt werden. Penicillin ab 0,002µg/ml, Tetracyclin ab 0,1µ/ml, Chloramphenicol ab 4µg/ml.

**Beispiel 4**

Testvorrichtung zum Nachweis von Antibiotika in Flüssigkeiten.

200 g einer Dispersion aus Vinylacetat und Vinylpropionat, 90 g einer Lösung von 30 g Polyvinylpyrrolidon, 10 g Polyäthylenglycol in loo ml eines 0,2 molaren Citratpuffers von pH 6,5 , 5 g Glucose, 7,2 g Polyoxyethylensorbitanmonoooleat, 10 Sporen von Bacillus subtilis ATTC 6o51 werden zu einer homogenen Masse verrührt.

Diese Filmrohmasse wird mit einer Schichtdicke von 100μ auf eine transparente Folie beschichtet, getrocknet und anschließend in 1 cm breite Bahnen geschnitten. Diese Filmbahnen werden gemeinsam mit einem Nährkarton, der mit den Nährstoffen für Antibiotika-Testmedien und Triphenyltetrazoliumchlorid imprägniert ist, in bekannter Weise, zu 1 cm breiten Teststreifen so verarbeitet, daß der Nährkarton auf eine Trägerfolie zu liegen kommt, der Sporenfilm mit der Filmseite auf dem Nährkarton liegt und beide mit einem Netz , das neben dem Nährkarton auf der Folie befestigt ist, festgehalten werden.

Mit der so hergestellten Testvorrichtung können Antibiotika ab den unten aufgeführten Konzentrationen über ihre Hemmwirkung nachgewiesen werden. Dazu werden die Teststreifen in die zu untersuchenden wäßrigen Lösungen eingetaucht und bei 37°C bebrütet. Bei Abwesenheit von Hemmstoffen verfärben sich die Testfelder tief rot, bei Anwesenheit von Hemmstoffen tritt bis zu den unten angegebenen Konzentrationen eine Aufhellung der Farbe ein. Bei höheren Hemmstoffkonzentrationen unterbleibt das Wachstum der Sporen völlig, es tritt keine Verfärbung des Testbezirkes ein.

**Tabelle 5**

Antibiotika mit Angaben zur minimalen Hemmkonzentration für Bacillus subtilis ATCC 6051 im Agarverdünnungstest(AVT) und im erfindungsgemäßen Verfahren.

| Antibiotika | AVT in μg/ml | Teststreifen in μg/ml |
|---|---|---|
| Penicillin G | o,oo2 | o,oo2 |
| Kanamycin | 4 | 4 |
| Tetracyclin | 4 | 1 |
| Polymyxin | 32 | 16 |
| Erythromycin | o,125 | o,32 |
| Gentamicin | o,125 | o,2 |
| Chloramphenicol | 2 | 2 |
| Cotrimoxazol | 8 | 8 |
| Ceftizoxim | 8 | 8 |

**Beispiel 5**

Testvorrichtung zum Nachweis von antibiotisch wirksamen Substanzen in Flüssigkeiten

450 g einer Dispersion aus Vinylacetat und Vinylpropionat, 230 g einer Lösung von: 80 g Dextran; 5,6 g Citronensäure; 3,8 g NaOH; 4,5 g 1,2-Propandiol in 155 ml Wasser; 10 g Glucose; 2 ml einer Suspension, die 2 x 10$^9$ Sporen von Bacillus subtilis enthält, werden homogen verrührt.

Diese Filmrohmasse wird mit einer Schichtdicke von 120μ auf eine transparente Folie beschichtet, getrocknet und anschließend in 1 cm breite Bahnen geschnitten.

Es wird durch Imprägnieren eines Filterkartons mit einer Nährbouillon, bestehend aus einem $\frac{1}{15}$ m Puffer von

2-(N-Morpholino)-äthansulfonsäure, des pH-Wertes 6,5, in dem Nährstoffe der üblichen Zusammensetzung und 0,04 % Triphenyltetrazoliumchlorid gelöst sind, ein Nährkarton hergestellt.

Dieser Nährkarton wird ebenfalls-in 1 cm breite Bahnen geschnitten. Sporenfilm und Nährkarton werden in bekannter Art und Weise zu 1 cm breiten Teststreifen so verarbeitet, daß der Nährkarton auf eine Trägerfolie zu liegen kommt, der Sporenfilm mit der Filmseite auf dem Nährkarton liegt und beide mit einem Netz, das neben dem Nährkarton auf der Folie befestigt ist,festgehalten werden.

Der oben genannte Puffer hat im Vergleich zu üblicherweise eingesetzten Puffern den Vorteil, daß Störungen auf das Wachstum der Sporen, die von hohen osmotischen Drucken des Harns herrühren, signifikant vermindert werden.

Mit der so hergestellten Testvorrichtung können Antibiotika ab den unten aufgeführten Konzentrationen über ihre Hemmwirkung nachgewiesen werden. Dazu werden die Teststreifen in die zu untersuchenden Körperflüssigkeiten eingetaucht und bei 37°C bebrütet. Bei Abwesenheit von Hemmstoffen verfärben sich die Testfelder tief rot, bei Anwesenheit von Hemmstoffen tritt bis zu den unten angegebenen Konzentrationen eine Aufhellung der Farbe ein. Bei höheren Hemmstoffkonzentrationen unterbleibt das Wachstum der Sporen völlig, es tritt keine Verfärbung des Testbezirks ein.

**Tabelle 6**

Antibiotika mit Angaben zu ihrer minimalen Hemmkonzentration, die mit dem erfindungsgemäßen Verfahren und die mit dem literaturbekannten Vergleichsverfahren (Blättchentest, Arztl. Lab. 10, 208 - 210 (1970)) aus Harnen gefunden werden.

| Antibiotikum | erfindungsgemäßes Verfahren ($\mu$ug/ml) | Vergleichsverfahren ($\mu$ug/ml) |
|---|---|---|
| Ampicillin | o,o16 | o,125 |
| Cefazolin | o,125 | 2 |
| Chloramphenicol | 2 | 8 |
| Fosfomycin | 256 | 256 |
| Gentamycin | 8 | 1 |
| Nitrofurantoin | 16 | 64 |
| Penicillin G | o,o16 | o,o31 |
| Pipemidsäure | 32 | 64 |
| Polymixin B | 512 | 512 |
| Tetracyclin | 1 | 8 |
| Trimethoprim | 16 | 32 |
| Cotrimoxazol | 2 | 128 |

**Patentansprüche**

1 Vorrichtung zum Nachweis von Mikroorganismen hemmenden Stoffen in einer flüssigen Probe, bestehend aus einem Nährsubstrat und einem Mikroorganismen enthaltendem Substrat, dadurch gekennzeichnet, däß das mikroorganismenhaltige Substrat ein aus einer Kunststoffdispersion hergestellter trockener Film ist, welcher Mikroorganismen in einer Menge von 10 - 10 pro g und wasserlösliche oder wasserquellbare, makromolekulare öffner sowie gegebenenfalls weitere Zusatzstoffe enthält, wobei das Mengenverhältnis von Kunststoff zu öffner 1000 : 1 bis 1 : 10 beträgt.

2. Vorrichtung nach Anspruch 1, dadurch gekennzeichnet, daß der Dispersionsfilm auf einem durchsichtigen Träger befestigt ist.

3. Vorrichtung nach Anspruch 2, dadurch gekennzeichnet, daß der Träger ein Gefäß ist, auf dessen Boden oder Wand der Film befestigt ist.

4. Vorrichtung nach Anspruch 3, dadurch gekennzeichnet, daß das Nährsubstrat als stabiles trockenes Gel bzw. als Lyophilisat enthalten ist.

5. Vorrichtung nach Anspruch 1 oder 2, dadurch gekennzeichnet, daß das Nährsubstrat aus einem schichtförmigen, mit den Nährsubstanzen imprägnierten saugfähigen Material besteht, der auf einem Träger befestigt und mit dem Dispersionsfilm bedeckt ist, welcher seinerseits an einem abdeckenden Träger befestigt ist.

6. Vorrichtung nach Anspruch 5, dadurch gekennzeichnet, daß das saugfähige Material Filterpapier oder ein Vlies ist.

## Claims

1. Device for the detection of substamces inhibiting micro-organisms in a liquid sample, consisting of a nutrient substrate and a micro-organism-containing substrate, characterised in that the micro-organismcontaining substrate is a dry film produced from a synthetic resin dispersion which contains micro-orgamisms in an amount of from 10 to 109 per g. and water-soluble or water-swellable, macrcmolecular openers, as well as possibly further additive materials, whereby the amount ratio of synthetic resin to opener amounts to 1000:1 to 1:10.

2. Device according to claim 1, characterised in that the dispersion film is fixed to a transparent carrier.

3. Device according to claim 2, characterised in that the carrier is a vessel on the bottom or walls of which is fixed the film.

4. Device according to claim 3, characterised in that the nutrient substrate is present as stable dry gel or as lyophilisate.

5. Device according to claim 1 or 2, characterised in that the nutrient substrate consists of a laminar absorbent material impregnated with the nutrient substances, which is fixed to a carrier and covered with the dispersion film, which, im turn, is fixed to a covering carrier.

6. Device according to claim 5, characterised in that the absorbent material is filter paper or a fleece.

## Revendications

1. Appareil pour la mise en évidence de substances inhibitoires pour micro-organismes dans un échantillon liquide, composé d'un substrat nutritif et d'un substrat contenant des micro-organismes, caractérisé en ce que le substrat contenant des micro-organismes est un film sec obtenu à partir d'une dispersion de matière synthétique, contenant des micro-organismes en une quantité de 10 à 109par g ainsi que des agents d'ouverture macromoléculaires solubles ou gonflants dans l'eau, ainsi qu'éventuellement d'autres additifs, le rapport de mélange entre matière synthétique et agents d'ouverture étant compris entre 1000/1 et 1/10.

2. Appareil selon la revendication 1, caractérisé en ce que le film de matière dispersée est fixé sur un support transparent.

3. Appareil selon la revendication 2, caractérisé en ce que le support est un recipient au fond ou à la paroi duquel est fixé le film.

4. Appareil selon la revendication 3, caractérisé en ce que le substrat nutritif est present sous la forme d'un gel sec et stable ou d'un lyophylisat.

5. Appareil selon la revendication 1 ou 2, caractérisé en ce que le substrat nutritif est une couche de matière absorbante imprégnée des substances nutritives, fixée sur un support et couverte du film de matière dispersée, qui à son tour est fixé à un support de couverture.

6. Appareil selon la revendication 5, caractérisé en ce que la matière absorbante est un papier filtre ou un voile.